# FASCICULE DE BREVET EUROPEEN

(11) **EP 0 533 896 B1**
(45) Date de publication et mention de la délivrance du brevet: **18.09.1996**
(21) Numéro de dépôt: 92909536.2
(22) Date de dépôt: 27.03.1992
(51) Int. Cl.: A61K 31/185, A61K 9/72

(54) **NOUVELLE COMPOSITION PHARMACEUTIQUE A BASE DE TAURINE POUR L'ADMINISTRATION PAR INHALATION**
ARZNEIMITTEL FÜR INHALATORISCHE ANWENDUNGEN BESTEHEND AUS TAURIN
INHALANT BASED ON TAURINE

(30) Priorité: 27.03.1991 FR 9103693
(43) Date de publication de la demande: 31.03.1993
(73) Titulaire: PROCTER & GAMBLE PHARMACEUTICALS FRANCE, 92201 Neuilly-sur-Seine (FR)
(72) Inventeur: COVARRUBIAS, Jésus, I-Florence (IT)
(74) Mandataire: L'Helgoualch, Jean
(86) Numéro de dépôt international: FR9200282
(87) Numéro de publication internationale: WO9217170

(56) Documents cités:
- EP-A- 0 307 788
- STN International Information Services, Base de Donnees: Chemical Abstracts, vol. 115, no. 9, numéro d'accès 92274m, Columbus, Ohio, US; & JP-A-03 068 549

## Description

La présente invention concerne une nouvelle forme d'administration de la taurine et de l'hypotaurine procurant une nouvelle indication thérapeutique, et plus particulièrement une application de la taurine et de l'hypotaurine par inhalation, notamment par administration sous forme d'aérosol, pour le traitement de l'asthme, ainsi que de diverses affections respiratoires liées à la pollution atmosphérique chez certains sujets hyper-réactifs.

La taurine, ou acide 2-aminoéthanesulfonique, est un acide aminé de faible toxicité, dont les propriétés ont été décrites dans de nombreuses publications. La taurine est peu utilisée comme médicament, et son utilisation en thérapeutique a été décrite dans la littérature uniquement pour le traitement de l'insuffisance cardiaque.

On connaît également quelques dérivés de la taurine dont les propriétés pharmacologiques ont été décrites, et par exemple, le brevet EP 307.788 décrit un amide de taurine présentant des propriétés mucolytiques, hépatoprotectrices, détoxifiantes et normolipémiantes. D'autres amides de taurine, utiles comme antagonistes de leucotriènes, sont décrits dans la demande de brevet JP-A-03.068549.

Depuis plusieurs années, de nombreuses études ont été menées sur les effets de la pollution atmosphérique, notamment par le dioxyde de soufre, le dioxyde d'azote, le monoxyde de carbone, les fumées organiques, l'ozone, etc, qui sont en partie responsables de maladies telles que des bronchites chroniques, l'amphysème, ou l'asthme. On a en particulier constaté que le développement de ces maladies était plus important dans les pays industrialisés soumis à une pollution atmosphérique importante, notamment dans les zones urbanisées où la pollution des gaz d'échappement de véhicules s'ajoute à celle des installations industrielles. On a aussi montré qu'une augmentation du taux de dioxyde d'azote dans l'air respiré par une population, provoque un accroissement du nombre de personnes atteintes d'une crise d'asthme.

D'autre part, on a montré que l'exposition prolongée de hamsters au dioxyde d'azote provoque une inflammation des bronchioles, des conduits alvéolaires, et des alvéoles péribronchiales, ainsi qu'une altération et une prolifération de cellules épithéliales. Ceci est probablement dû au fait que le dioxyde d'azote est un agent oxydant, de même que le monoxyde de carbone et le dioxyde de soufre, qui agit sur la membrane cellulaire par un mécanisme qui n'est toutefois pas clairement expliqué aujourd'hui.

On sait que l'administration de certains composés, exerçant une action d'agent anti-oxydant ou de séquestrant de radicaux libres, permet de procurer une certaine protection contre ces affections. Par exemple, quelques effets favorables ont été observés dans des essais sur des modèles de laboratoire, avec de la vitamine E (alpha-tocophérol), du glutathion, le diméthylsulfoxyde ou de la N-acétylcystéine. Cependant les problèmes liés à la toxicité ne sont pas résolus, et en particulier, si la vitamine E agit comme piège à radicaux libres, elle ne procure pas de protection efficace contre les agents oxydants tels que le dioxyde d'azote ou de soufre, et ne renforce pas la barrière épithéliale des bronches. Ainsi, ni la vitamine E, ni d'autres pièges à radicaux libres tel que le DMSO ne peuvent modifier l'hyper-réactivité de certains sujets, et aucune protection satisfaisante n'est donc obtenue.

L'utilisation de sels d'acide cromoglycique, tels que le sel disodique, a également été proposée pour le traitement de l'asthme d'origine allergique. Par exemple, l'administration de cromoglycate de sodium entraîne une stabilisation de l'épithélium au niveau des bronches, mais ne procure pas de protection contre les agents polluants oxydants et les initiateurs de radicaux libres.

De nombreuses recherches ont été faites pour tenter de mettre au point des composés ou compositions qui soient à la fois des pièges à radicaux libres et des antioxydants, et qui stabilisent et protègent l'épithélium des alvéoles et des bronches, afin de procurer une protection satisfaisante des voies respiratoires contre les maladies liées à la pollution.

On a maintenant constaté que l'administration de taurine ou d'hypotaurine par innalation, notamment sous forme d'aérosol, pouvait constituer un excellent traitement de prévention des maladies des voies respiratoires, plus particulièrement un traitement de l'asthme, ainsi que de certaines maladies liées à la pollution atmosphérique indiquées ci-dessus, telles que la bronchite chronique et l'amphysème, tandis que les autres formes d'administration sont inefficaces.

L'invention a donc pour objet une nouvelle application de la taurine et de l'hypotaurine sous forme d'aérosol, pour le traitement des affections des voies respiratoires, plus particulièrement pour le traitement préventif de l'asthme ainsi que de maladies liées à la pollution atmosphérique. Ainsi, la nouvelle composition pharmaceutique conforme à la présente invention contient de la taurine ou de l'hypotaurine en combinaison avec des moyens pour son administration par inhalation.

Les expérimentations cliniques effectuées ont montré que l'administration de taurine par inhalation, sous forme d'aérosol, entraîne une diminution significative des bronchospasmes induits par nébulisation d'eau distillée, et procure notamment un effet protecteur contre les réactions asthmatiques induites.

Les résultats de ces expérimentations montrent que la nouvelle composition à base de taurine ou d'hypotaurine selon l'invention, administrée en aérosol, peut être utilisée de manière inattendue dans une nouvelle indication, c'est-à-dire pour le traitement de base et préventif des affections des voies respiratoires, plus particulièrement de l'asthme avec une efficacité satisfaisante, sans entraîner d'effet secondaire néfaste.

Ces résultats peuvent être obtenus conformément à la présente invention avec la taurine (acide 2-amino-éthane sulfonique) comme avec l'hypotaurine (acide 2-amino-éthane sulfinique) précurseur métabolique de la taurine, administrés par inhalation, et de préférence sous forme d'aérosol, tandis qu'aucun résultat favorable n'est observé dans le cas d'une administration sous forme de soluté buvable ou par injection intraveineuse.

Conformément à la présente invention, la taurine ou l'hypotaurine est en solution dans un solvant approprié, et est présentée de préférence en aérosol, en combinaison avec un gaz propulseur de type usuel, dans un récipient adapté, permettant de créer un nébulisat qui est projeté dans les voies respiratoires, c'est-à-dire dans la bouche et la trachée artère du patient recevant le traitement.

Le solvant utilisé pour les solutions de taurine est choisi parmi les solvants usuels pharmaceutiquement acceptables, mais on utilise de préférence la taurine sous forme de solution aqueuse.

La concentration de taurine ou d'hypotaurine en solution peut être comprise entre 5 g/l et 50 g/l de solution, et de préférence entre 15 g/l et 25 g/l.

Le gaz propulseur peut être par exemple un fréon du type couramment utilisé dans les aérosols.

Les doses de taurine administrées par aérosol suivant l'invention sont d'environ 5 mg à 20 mg par jour et par kg de poids, les doses unitaires étant généralement comprises entre 100 mg et 500 mg, et le nombre d'inhalations par jour étant habituellement compris entre 3 et 5 pour un sujet adulte.

L'efficacité de la composition à base de taurine ou d'hypotaurine selon la présente invention a été vérifiée par une expérimentation réalisée sur 9 patients d'age compris entre 10 et 40 ans, souffrant d'asthme chronique, dans les conditions indiquées ci-après.

Des crises d'asthme sont provoquées par le test d'inhalation d'eau distillée nébulisée par ultrasons, et les valeurs spirométriques des patients sont mesurées avant et immédiatement après l'inhalation d'eau distillée. Le même test est répété après un délai d'au moins trois à quatre jours, mais 30 minutes environ après inhalation de 250 mg de taurine en aérosol, conformément à la présente invention, et les mesures spirométriques sont à nouveau effectuées, puis les résultats sont traités statistiquement.

On constate que le premier test, sans inhalation de taurine en aérosol, entraîne une diminution significative des paramètres spirométriques des patients montrant une instabilité bronchiales caractéristique des sujets asthmatiques. Le paramètre FVC est en effet ramené de 3,32 à 2,64. Au contraire, après inhalation de taurine, le même paramètre n'est pas modifié de manière significative (3,47 avant l'inhalation d'eau distillée, et 3,35 après).

La même expérience est répétée avec deux groupes de patients, le premier groupe recevant de la taurine en aérosol, conformément à l'invention (dose administrée : 125 mg), tandis que le deuxième groupe reçoit de la taurine sous forme de solution buvable (dose administrée : 500 mg).

On observe alors que seul le premier groupe présente une résistance au test de l'inhalation d'eau distillée déclenchant le bronchospasme.

Ces résultats montrent que la composition à base de taurine ou d'hypotaurine conforme à la présente invention, administrée sous forme d'aérosol, permet d'agir efficacement comme traitement préventif contre les crises d'asthme, et d'une manière générale, contre les affections respiratoires liées à la pollution atmosphérique.

Une étude comparative est effectuée dans les mêmes conditions, en comparant les résultats obtenus par inhalation de taurine conformément à l'invention, d'une part, et par administration de cromoglycate de sodium en aérosol suivant une technique usuelle, d'autre part. On constate alors une meilleure efficacité de l'inhalation de taurine.

## Revendications

1. Composition pharmaceutique pour le traitement de l'asthme et des affections des voies respiratoires, caractérisée en ce qu'elle contient de la taurine ou de l'hypotaurine en combinaison avec des moyens pour son administration par inhalation.

2. Composition selon la revendication 1, caractérisée en ce que elle comprend des moyens pour l'administration de la taurine ou de l'hypotaurine en aérosol.

3. Composition selon l'une quelconque des revendications précédentes, caractérisée en ce que elle comprend de la taurine ou de l'hypotaurine en solution dans un solvant approprié, en combinaison avec un gaz propulseur.

4. Composition selon la revendication 3, caractérisée en ce que la concentration de la taurine est comprise entre 5 g/l et 50 g/l de solvant.

5. Composition selon l'une quelconque des revendications 3 et 4, caractérisée en ce que la taurine est en solution aqueuse.

6. Utilisation de la taurine ou de l'hypotaurine pour la préparation d'une composition pharmaceutique pour l'administration par inhalation, pour le traitement de l'asthme et des affections des voies respiratoires.

## Claims

1. Pharmaceutical composition for the treatment of asthma and diseases of respiratory ducts, characterized in that it contains taurine or hypotaurine in combination with means for the administration thereof by inhalation.

2. Composition according to claim 1, characterized in that it comprises means for the administration of taurine or hypotaurine as aerosol.

3. Composition according to any of the preceding claims, characterized in that it comprises taurine or hypotaurine in solution in an appropriate solvent, in combination with a gaseous propellent.

4. Composition according to claim 3, characterized in that the taurine concentration is comprised between 5 g/l and 50 g/l of solvent.

5. Composition according to any of claims 3 and 4, characterized in that taurine is in aqueous solution.

6. The use of taurine or hypotaurine for the preparation of a pharmaceutical composition for administration by inhalation for the treatment of asthma and diseases of respiratory ducts.

## Patentansprüche

1. Pharmazeutische Zusammensetzung zur Behandlung von Asthma und Atemwegserkrankungen, dadurch gekennzeichnet, daß sie Taurin oder Hypotaurin in Kombination mit Mitteln für dessen Verabreichung durch Inhalieren enthält.

2. Zusammensetzung nach Anspruch 1, dadurch gekennzeichnet, daß sie Mittel zur Verabreichung von Taurin oder Hypotaurin als Aerosol umfaßt.

3. Zusammensetzung nach einem der vorangegangenen Ansprüche, dadurch gekennzeichnet, daß daß sie Taurin oder Hypotaurin in Lösung in einem geeigneten Lösungsmittel in Kombination mit einem Treibgas enthält.

4. Zusammensetzung nach Anspruch 3, dadurch gekennzeichnet, daß die Konzentration des Taurins zwischen 5 g/l und 50 g/l Lösungsmittel liegt.

5. Zusammensetzung nach einem der Ansprüche 3 oder 4, dadurch gekennzeichnet, daß das Taurin in wäßriger Lösung vorliegt.

6. Verwendung von Taurin oder Hypotaurin zur Herstellung einer pharmazeutischen Zusammensetzung zur Verabreichung durch Inhalieren, zur Behandlung von Asthma und Atemwegserkrankungen.
